(19) Europäisches Patentamt
European Patent Office
Office européen des brevets

(11) Veröffentlichungsnummer: **0 072 435**
**B1**

(12) **EUROPÄISCHE PATENTSCHRIFT**

(45) Veröffentlichungstag der Patentschrift: **18.12.85**

(51) Int. Cl.⁴: **C 08 G 77/38,** C 08 G 77/26, C 08 G 77/50

(21) Anmeldenummer: **82106356.7**

(22) Anmeldetag: **15.07.82**

(54) **Polymere Metall-Amin-Komplexverbindungen, Verfahren zu ihrer Herstellung und Verwendung.**

(30) Priorität: **13.08.81 DE 3131954**

(43) Veröffentlichungstag der Anmeldung:
**23.02.83 Patentblatt 83/8**

(45) Bekanntmachung des Hinweises auf die Patenterteilung:
**18.12.85 Patentblatt 85/51**

(84) Benannte Vertragsstaaten:
**AT BE CH DE FR GB IT LI NL**

(56) Entgegenhaltungen:
**DE - A - 3 029 599**
**DE - A - 3 120 195**
**DE - A - 3 120 214**
**US - A - 4 292 253**

(73) Patentinhaber: **Degussa Aktiengesellschaft, Weissfrauenstrasse 9, D-6000 Frankfurt am Main 1 (DE).**

(72) Erfinder: **Panster, Peter, Dr., Im Lochseif 8, D-6458 Rodenbach 2 (DE)**
Erfinder: **Kleinschmit, Peter, Dr., Wildaustrasse 19, D-6450 Hanau 9 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft neue polymere Koordinationsverbindungen von Metallen der VI., VII., VIII., und I. Nebengruppe des Periodensystems mit polymeren Siliciumsubstituierten Aminen als Liganden, Verfahren zu ihrer Herstellung und die Verwendung dieser heterogenen Metallkomplexe als Katalysatoren.

Bekanntlich beeinhaltet der Einsatz sog. homogener Katalysatoren in der Technik insbesondere die Probleme der Katalysatorabtrennung und -recyclierung, der Rückgewinnung des meist wertvollen Metalls, der überwiegend nur kurzen Standzeit dieser Vertreter und der oft starken Korrosion der Produktionsanlage durch die teilweise salzartigen Metallverbindungen. Intensive Bemühungen gelten deshalb bereits seit einiger Zeit dem Ziel, diese Nachteile homogener Katalysatoren zu umgehen und die Vorteile der homogenen und heterogenen Katalysatoren in sog. heterogenisierten Katalysatoren zu vereinigen. Diese bestehen aus prinzipiell löslichen Metalleinheiten, die über kovalente, ionogene oder adsorptive Wechselwirkungen an Träger gebunden werden. Der Stand der Technik auf diesem Gebiet wurde bereits in mehreren Übersichtsartikeln, z.B. auch durch R.H. Grubbs in CHEMTECH Aug. 1977, Seite 512 oder durch D.D. Whitehurst in CHEMTECH Jan. 1980, Seite 44 zusammengefasst. Als Trägermaterialien eingesetzt wurden dabei bisher vor allem organische Polymere, die überwiegend erst nach einer geeigneten Modifizierung, z.B. im Fall von Polystyrol durch primäre Einführung von Chloromethylgruppen, zur Fixierung löslicher Metallkomplex-Katalysatoren befähigt sind. Die Heterogenisierung von Metallkomplexen unter Verwendung organischer Polymerer als Träger ist z.B. von R.H. Grubbs und L.C. Kroll in J. Amer. Chem. Soc. 93, 3062 (1971), von M. Čapka et al. in Tetrahedron Letters 1971, 4787 oder auch in der britischen Patentschrift 1 277 737 beschrieben.

Im allgemeinen weisen die als Trägermaterialien verwendeten organischen Polymeren jedoch keineswegs die Qualitäten auf, die einen guten Katalysatorträger auszeichnen sollten, denn sie besitzen keine festliegende Struktur, ihre Konformation und damit Oberfläche sowie Volumen der einzelnen Partikel hängen stark von äusseren Parametern, wie Temperaturen, Druck und Lösungsmittel ab. Ein Schwellen der Träger im verwendeten Solvens ist stets notwendig, um ein Vordringen der Reaktanten zu den Katalysezentren zu ermöglichen und die Reaktionsgeschwindigkeit nicht diffusionskontrolliert werden zu lassen. Die hohe Mobilität der Matrix gestattet auch das Zusammenkommen fixierter Metalleinheiten, so dass eine unerwünschte Bildung katalytisch inaktiver Mehrkernkomplexe ermöglicht wird und darüber hinaus trägergebundene, unkoordinierte Liganden das katalytisch aktive Metallzentrum blockieren können (vgl. G. Strukul, P. D'Olimpio, M. Bonivento, F. Pinna und M. Graziani in J. Mol. Catal. 2, 179 (1977)). Es kann auch der Fall eintreten, dass eine Auflösung der polymeren organischen Matrix in solchen Lösungsmitteln erfolgt, die an sich für die katalytische Reaktion vorteilhaft wären.

Demgegenüber besitzen anorganische Polymersysteme, wie z.B. gefällte oder pyrogene Kieselsäuren, eine festliegende Struktur und eine bei weitem höhere Temperatur- sowie Alterungsbeständigkeit und ausserdem sitzen die fixierten Metalleinheiten leicht zugänglich an der Oberfläche. Es ist daher verständlich, dass anorganische Träger bereits zur Fixierung homogener Katalysatoren verwendet wurden, wie dies z.B. in der US-PS 4 083 803, in der DE-OS 20 62 351 oder von H.H. Brintzinger et al. in J. Organomet. Chem. 148, 73 (1978) beschrieben ist. Einen gravierenden Nachteil besitzen anorganische Trägermaterialien allerdings insofern, als die Anzahl der Hydroxylgruppen, über die eine Bindung zum Liganden bzw. zum Metallatom vollzogen werden kann, relativ gering ist, so dass keine hohe Beladung mit Liganden bzw. Metallatomen erfolgen kann und viel Trägerballast mit dem Katalysator herumgeschleppt wird.

Vor kurzer Zeit konnte, wie in den DE-A-28 34 691 und 30 29 599 (beide nachveröffentlicht) beschrieben, ein neues Prinzip gefunden werden, nach dem homogene Sulfid- und Phosphin-Komplexkatalysatoren der Metalle Rhodium, Iridium und Ruthenium ohne Verwendung eines Trägers heterogenisiert werden können. Der polymere Träger, aufgebaut aus intra- und intermolekular gebildeten Siloxaneinheiten, wird dabei durch Hydrolyse und Kondensation der an den Liganden vorhandenen Trialkoxi-, Triphenoxi- oder Trihalosilyleinheiten erzeugt.

Er zeigt erwartungsgemäss auch die vorstehend genannten guten Eigenschaften anorganischer Träger und lässt sich darüber hinaus quasi massgeschneidert herstellen, z.B. in bezug auf die so wichtigen Aspekte, dass mehr oder weniger Liganden als nach der Stöchiometrie des zu heterogenisierenden Komplexes erforderlich sind oder sogenannte Vernetzer, über die eine Steuerung der Katalysezentrendichte im Feststoff möglich ist, und auch sog. Aktivatoren oder Cokatalysatoren in die Matrix eingebaut werden können. Gegenüber Systemen auf Basis anorganischer Träger weisen diese kieselsäureartigen polymeren Systeme jedoch vor allem die Vorteile auf, eine höhere Metallkonzentration bei gleichem Ligand : Metall-Verhältnis besitzen zu können, präparativ einfacher zugänglich und durch den stark hydrophoben Charakter der Matrix resistenter gegenüber Hydroxiden zu sein.

Es konnte nun gefunden werden, dass nach dem Konzept der Polykondensation von Liganden, welche mit trifunktionellem Silicium substituiert sind, nicht nur polymere Rhodium-, Iridium- und Ruthenium-Sulfid- bzw. Phosphin-Komplexe, sondern auch eine breite Palette für die Katalyse sehr interessanter, vielseitig einsetzbarer und aktiver polymerer Amin-Komplexe des Molybdäns, Wolframs, Mangans, Rheniums und der Metalle der VIII. und I. Nebengruppe des Periodensystems zugänglich sind. Dabei kommt den Rhodium-, Iridium-, Ruthenium-, Palladium- und Platin-Verbindungen sowie den Kobalt-Verbindungen besondere Bedeutung zu. Auch bei diesen heterogenisierten Komplexkatalysatortypen konnten die bei vergleichbaren monomeren Systemen vorhandenen spezifischen und ligandentypischen Eigenschaften erhalten werden. Ebenso, wie schon bei den Sulfid- und Phosphinsystemen, weist auch hier die polymere Matrix die vorstehend erwähnten vorzüglichen Eigenschaften auf.

Die neuen polymeren Aminkomplexe des Molybdäns, Wolframs, Mangans, Rheniums und der Metalle der VIII. und I. Nebengruppe des Periodensystems mit kieselsäureartiger Struktur sind dadurch gekennzeichnet, dass am metallischen Zentralatom wenigstens ein Amin der allgemeinen Formel

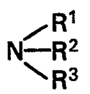 (1)

koordinativ gebunden sind, ein atomares Metall : Stickstoff-Verhältnis von 1:1 bis $1:10^6$ vorliegt, die gegebenenfalls noch freien Koordinationsstellen am Metallatom mit anderen Elektronenpaar-Donatoren, wie z.B. Kohlenmonoxid, Stickoxid, Triphenylphosphin, Triphenylarsin, Phosphit, ein sekundäres oder tertiäres Alkylamin mit 1 bis 5 C-Atome enthaltenden linearen oder verzweigten Alkylgruppen, Benzylamin, Dialkylsulfid, Olefin, Diolefin, Acetylen, Nitril, Isonitril, Cyanat, Isocyanat oder Wasser (Kristallwasser) besetzt sind und ein erforderlicher Ladungsausgleich mit einem anorganischen oder organischen Anion, wie Chlorid-, Bromid-, Jodid-, Nitrat-, Sulfat-, Phosphat-, Acetylacetonat-, Acetat-, Trifluoracetat-, Trichloracetat-, Propionat-, Methylat-, Ethylat-, Propylat-, Butylat-, Phenylat-, Perchlorat-, Tetraphenylborat-, Hexafluorophosphat-, Methyl-, Ethyl-, Propyl-, Butyl-, Phenyl- oder Perfluorphenylion, gegebenenfalls unter vollständigem oder partiellem Ersatz solcher Anionen durch Hydridion, vollzogen ist, wobei in der Formel (1)

$R^1$ und $R^2$ für eine Gruppe der allgemeinen Formel

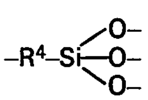 (2)

stehen, $R^4$ eine lineare oder verzweigte Alkylengruppe mit 1 bis 10 C-Atomen oder eine Cycloalkylengruppe mit 5 bis 8 C-Atomen bedeutet oder für Einheiten

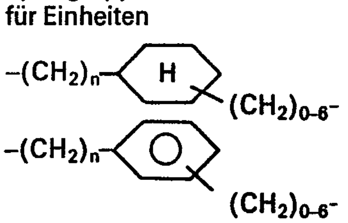 bzw.

steht, wobei n 1 bis 6 stickstoffständige Methylengruppen sind, $R^1$ und $R^2$ gleich oder verschieden sein können und die freien Valenzen der Sauerstoffatome entweder durch Siliciumatome weiterer Gruppen der Formel (2) und/oder durch vernetzende Brückenglieder

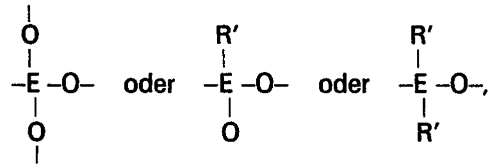

wobei E für Silicium, Titan oder Zirkonium steht, bzw. durch

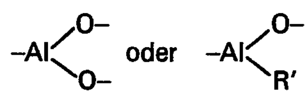

abgesättigt sind, wobei R' eine Methyl- oder Ethylgruppe ist und das Verhältnis zwischen den Siliciumatomen in (2) zu den Brückenatomen Silicium, Titan, Zirkonium, Aluminium 1:0 bis 1:10 betragen kann,

$R^3$ dieselbe allgemeine Bedeutung wie $R^1$ und $R^2$ hat oder für Wasserstoff, eine lineare oder verzweigte Alkylgruppe mit 1 bis 10 C-Atomen oder eine Cycloalkylgruppe mit 5 bis 8 C-Atomen oder die Benzylgruppe steht.

Die monomeren Vorstufen der Amine der Formel (1) und auch die polymeren Amine selbst können nach gängigen Verfahren, beispielsweise nach den Anweisungen der DE-A- 31 20 214 (nachveröffentlicht), welcher die EP-A-0 066 074 (nachveröffentlicht) entspricht, hergestellt werden. Die Zusammensetzung der monomeren Vorstufen lässt sich beispielhaft durch Formeln, wie

$(H_3C)N[CH_2CH_2CH_2Si(OC_2H_5)_3]_2$,
$(C_6H_5CH_2)N[CH_2CH_2CH_2Si(OC_2H_5)_3]_2$,
$(H_3C)N[CH_2Si(OC_2H_5)_3]_2$,
$N[CH_2CH_2CH_2Si(OC_2H_5)_3]_3$,

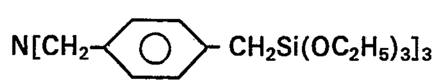

und die Zusammensetzung der entsprechenden polymeren Amine durch Formeln, wie

$(H_3C)N(CH_2CH_2CH_2SiO_{3/2})_2$,
$(C_6H_5CH_2)N(CH_2CH_2CH_2SiO_{3/2})_2$,
$(H_3C)N(CH_2SiO_{3/2})_2$,
$N(CH_2CH_2CH_2SiO_{3/2})_3$,

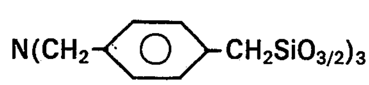

beschreiben.

Der stöchiometrische Aufbau der unter Verwendung der Aminliganden der allgemeinen Formel (1) hergestellten polymeren Übergangsmetallkomplexe kann im Fall des Molybdäns und Wolframs durch Formeln, wie

$MoX_4L_{1-10^6}$, $MoX_5L_{1-10^6}$, $Mo(CO)_{3-5}L_{1-10^6}$,
$WX_4L_{1-10^6}$, $WX_6L_{1-10^6}$, $W(CO)_{3-5}L_{1-10^6}$,

im Fall des Mangans und Rheniums durch Formeln wie

$MnXL_{1-10^6}$, $MnX_2L_{1-10^6}$, $ReX_3L_{1-10^6}$,
$ReX_4L_{1-10^6}$, $ReX_5L_{1-10^6}$, $ReX_6L_{1-10^6}$,

im Fall der Metalle der VIII. Nebengruppe des Periodensystems durch Formeln, wie

$FeXL_{1-10^6}$, $FeX_2L_{1-10^6}$, $FeX_3L_{1-10^6}$,
$Fe(CO)_{3-4}L_{1-10^6}$,
$CoXL_{1-10^6}$, $CoX_2L_{1-10^6}$, $CoX_3L_{1-10^6}$,
$Co_2(CO)_{6-7}L_{1-10^6}$,
$NiXL_{1-10^6}$, $NiX_2L_{1-10^6}$, $Ni(CO)_{2-3}L_{1-10^6}$,
$RuX_2L_{1-10^6}$, $RuX_3L_{1-10^6}$,
$RhXL_{1-10^6}$, $RhX_2L_{1-10^6}$, $RhX_3L_{1-10^6}$,
$PdXL_{1-10^6}$, $PdX_2L_{1-10^6}$, $PdX_4L_{1-10^6}$,
$OsX_4L_{1-10^6}$, $OsHXL_{1-10^6}$,
$IrXL_{1-10^6}$, $IrX_3L_{1-10^6}$,
$PtX_2L_{1-10^6}$, $PtX_4L_{1-10^6}$,

und im Fall der Metalle der I. Nebengruppe durch Formeln wie

$CuXL_{1-10^6}$, $CuX_2L_{1-10^6}$, $AgXL_{1-10^6}$, $AuXL_{1-10^6}$,
$AuX_3L_{1-10^6}$,

beschrieben werden, wobei L mindestens einen Liganden der Formel (1) darstellt, ansonsten für einen anderen Elektronenpaar-Donator steht und X ein einwertiges, zweiwertiges oder dreiwertiges Anion bedeutet.

Neben den zur engeren Koordinationssphäre des Metalls gehörenden Liganden L der Formel (1) können weitere unkoordinierte Liganden L der Formel (1) in der gegebenenfalls Vernetzerhaltigen polymeren Matrix vorhanden sein, und zwar maximal soviel, dass ein atomares Metall : Stickstoff-Verhältnis von $1:10^6$ vorliegt.

Darüber hinaus können auf dem polymeren Amin verschiedene Komplextypen verschiedener Metalle nebeneinander vorliegen (maximal 16), die sehr interessante und aktive Multimetall-Katalysatorsysteme darstellen.

Bevorzugt in der grossen Palette der herstellbaren neuen Metallkomplex-Systeme sind insbesondere die polymeren Komplexverbindungen, in denen X für Chlorid, Bromid, Jodid, Nitrat, Acetat, Sulfat, Carbonat, Phosphat, Acetylacetonat oder Hydrid und L ausschliesslich für Liganden der Formel (1) steht.

Die anderen vorstehend aufgeführten Komplextypen können teilweise erst nach einer chemischen Modifizierung der Ausgangspolymeren erhalten werden, die unter dem Aspekt einer weiteren Verbesserung der Aktivität oder Selektivität des heterogenisierten homogenen Katalysators durchgeführt werden kann oder muss. Diese Modifizierung besteht entweder in einer Erniedrigung der Oxidationsstufe des Metallatoms, in der teilweisen oder vollständigen Substitution der Anionen X durch andere Anionen oder in der zusätzlichen Einführung weiteter Anionen oder nicht matrixgebundener Liganden, wie z.B Triphenylphosphin. In der Praxis bedeutet das eine Umsetzung der polymeren Metallkomplexsysteme mit $H_2$, CO oder $H_2$ plus CO oder mit Reduktionsmitteln oder mit Lewissäuren oder die Einführung eines anderen Anions oder die Reaktion mit zusätzlichen Liganden bei Gesamtdrukken von 1 bis 3000 bar und Temperaturen von −100 bis 350 °C.

Als gängige Reduktionsmittel können Formaldehyd, Hydrazin, Alkali- oder Erdalkalimetallborhydride, Boranverbindungen, Aluminiumhydride, Aluminiumalkyle bzw. auch nur Alkohole verwendet werden.

Zur Einführung eines anderen Anions können Verbindungen wie Alkalimetallalkylate (z.B. Alkalimetallmethylat, -ethylat-, -propylat), Alkalimetallphenylate oder Alkalimetallalkyle oder -phenyle oder Natriumacetat, -acetylacetonat oder -jodid eingesetzt werden.

Für eine Reaktion mit zusätzlichen Liganden können Verbindungen wie Triphenylphosphin, -arsin, sekundäre oder tertiäre Alkylamine mit 1 bis 5 C-Atome enthaltenden linearen oder verzweigten Alkylgruppen, Benzylamin, Dialkylsulfide oder auch Olefine bzw. Diolefine eingesetzt werden.

Gegenstand der Erfindung ist auch ein Verfahren zur Herstellung dieser polymeren, in organischen Lösungsmitteln schwerlöslich bzw. unlöslichen Metall-Amin-Komplexverbindungen. Es ist im einzelnen dadurch gekennzeichnet, dass man ein bereits polymeres, gegebenenfalls Vernetzer der genannten Art enthaltendes Amin der Formel (1) mit zumindest teilweise gelösten, gegebenenfalls Chlorid, Bromid, Jodid, Nitrat, Acetat, Sulfat, Carbonat, Phosphat, Acetylacetonat oder Hydrid und/oder gegebenenfalls Liganden, wie Kristallwasser, Kohlenmonoxid, Amin, Triphenylphosphin, Phospit, Sulfid, Olefin, Acetylen, Nitril, Isonitril, Cyanat oder Isocyanat enthaltenden Verbindungen von einem oder mehreren der vorgesehenen Metalle, gegebenenfalls unter Verdrängung eines oder mehrerer Liganden, bei Raumtemperatur oder erhöhter Temperatur bis 350 °C, bei Normaldruck oder einem Überdruck, welcher der Summe der Partialdrucke der einzelnen Komponenten der Reaktionsmischung bei der jeweiligen Temperatur entspricht, umsetzt, den metallhaltigen polymeren Feststoff anschliessend durch Destillieren, Filtrieren, Zentrifugieren und/oder Dekantieren von der flüssigen Phase abtrennt, gegebenenfalls mit Wasser oder einem organischen Lösungsmittel wäscht bzw. extrahiert, nachfolgend bei Temperaturen von Raumtemperatur bis 200 °C trocknet, gegebenenfalls über einen Zeitraum von 1 Stunde bis 4 Tagen bei Temperaturen von 200 bis 400 °C tempert, anschliessend gegebenenfalls mahlt und/oder klassifiziert.

Die Auswahl der Lösungsvermittler oder auch Lösungsvermittlergemische, die für diese Umsetzung des polymeren Amins mit den Metallverbindungen in Frage kommen, ist nicht besonders kritisch, denn es eignen sich alle Lösungsvermittler, welche die Metallkomponente wenigstens teilweise zu lösen imstande sind und ausserdem deren Fixierung nicht derart behindern, dass sie starke koordinative Wechselwirkungen ausüben oder reduzierend bzw. oxidierend wirken. Im Falle der in-situ-Darstellung des polymeren Amins, welche in der DE-A- 31 20 214 beschrieben ist, sollte von vornherein ein Lösungsvermittler, wie z.B. ein Alkohol, verwendet werden, der im Gemisch mit Wasser auch für die weitere Umsetzung mit der Metallkomponente eingesetzt werden kann. Geeignete Flüssigkeiten für das Reaktionsmedium sind im allgemeinen Wasser, Methanol, Ethanol, n- und i-Propanol, n-, i- und t-Butanol, n-Pentanol, Dioxan, Nitromethan, Nitrobenzol, Ethylenglycolmonomethylether, Ethylenglycoldimethylether, Diethylenglycoldimethylether, Ethylenglycol, Aceton, Tetrahydrofuran, Dimethylformamid, Dimethylsulfoxid, Benzol, Toluol, Cyclohexan, Methylcylohexan, n-Hexan, Chloroform, Methylenchlorid oder Gemische aus diesen. Bevorzugt sind jedoch meist polare Lösungsmittel, wie Alkohole oder Alkohol/Wasser-Gemische.

Nach dem erfindungsgemässen Verfahren lassen sich auch polymere Multimetall-Amin-Komplexe, das heisst gemischte polymere, schwer- bzw. unlösliche Verbindungen von zwei oder mehreren der Metalle Molybdän, Wolfram, Mangan, Rhenium und der Metalle der VIII. und I. Nebengruppe des Periodensystems herstellen. Dabei kann nach einer zweckmässigen Ausgestaltung des erfindungsgemässen Verfahrens ein polymeres, gegebenenfalls Vernetzer enthaltendes Amin der Formel (1) gleichzeitig oder nacheinander mit verschiedenen, zumindest teilweise gelösten Verbindungen der vorgesehenen Metalle, wie beschrieben, umgesetzt werden, wobei der metallhaltige Feststoff, gegebenenfalls nach jeder Umsetzung, durch Destillieren, Filtrieren und/oder Dekantieren von der flüssigen Phase abgetrennt, gewaschen bzw. extrahiert, getrocknet und gegebenenfalls getempert wird.

Zur Gewinnung polymerer Multimetall-Amin-Komplexe kann also die Fixierung von zwei oder mehreren Verbindungen zweier oder mehrerer Metalle nebeneinander an das gleiche polymere Amin entweder durch gleichzeitige Reaktion des polymeren Amins mit den Metall-Ausgangsverbindungen unter den vorgestellten Bedingungen erfolgen oder man arbeitet in mehreren hintereinander geschalteten Reaktionsschritten, wobei der polymere Feststoff jeweils den genannten Aufarbeitungsmassnahmen teilweise oder vollständig unterworfen werden kann. Das erhaltene Multimetall-System kann am Ende einer Synthese entweder noch im Reaktionsmedium seiner Verwendung zugeführt werden oder es wird erst abgetrennt, gewaschen bzw. extrahiert, getrocknet, gegebenenfalls getempert, gemahlen und/oder klassifiziert, wobei unter Umständen eine oder auch mehrere dieser Massnahmen ausgelassen werden können.

Die polymeren, ein oder mehrere Metalle enthaltenden Komplexverbindungen können weiter modifiziert werden indem sie, vorzugsweise suspendiert in einem Lösungsmittel, mit $H_2$, CO oder $H_2$ plus CO oder mit Reduktionsmitteln oder mit Lewissäuren oder durch Einführung eines anderen Anions oder durch Reaktion mit zusätzlichen Liganden bei Gesamtdrucken von 1 bis 3000 bar und Temperaturen von −100 bis 350 °C ein- oder mehrmalig nachbehandelt werden. Diese Modifizierung kann je nach dem gewählten Behandlungsagens schon im Reaktionsmedium oder nach Durchführung von einer oder mehreren der Behandlungsstufen Isolierung, Trocknung, Temperung, durchgeführt werden. Selbst während des Einsatzes der Komplexe für eine bestimmte Anwendung kann die Modifizierung vorgenommen werden.

Ein Modifizierungsgang kann beispielsweise darin bestehen, dass der zu behandelnde Feststoff unter kräftiger Durchmischung, gegebenenfalls in Anwesenheit eines Lösungsvermittlers, mit einer stöchiometrischen oder überschüssigen Menge einer Reaktionskomponente, welche in Reinsubstanz vorliegt und gasförmig oder gelöst sein kann, bei den vorgesehenen Druck- und Temperaturbedingungen behandelt wird.

Soll eine Modifizierung an nur einer Ausgangskomponente von polymeren Multimetall-Amin-Komplexen durchgeführt werden, bringt man diese Ausgangskomponente am besten zuerst auf das polymere Amin auf und führt im Anschluss daran, gegebenenfalls nach vorheriger Isolierung, Trocknung und einem Wechsel des Lösungsvermittlers, die entsprechende Behandlung durch, ehe weitere Metallverbindungen auf den polymeren Aminträger aufgebracht werden. In einigen Fällen kann jedoch auch eine gemeinsame Behandlung des bereits vollständig vorliegenden Multimetall-Ausgangssystems erfolgen.

Ein weiteres, vor allem wegen der leichten Reduzierbarkeit vieler Metallsysteme nicht in derart breitem Rahmen anwendbares Verfahren zur Herstellung der neuen polymeren Metall-Amin-Komplexe besteht darin, dass man unter Verwendung einer monomeren Vorstufe eines Amins nach Formel (1) ein monomeres Metall-Amin-Komplexsystem, in welchem gegebenenfalls noch weitere Elektronenpaar-Donatoren am Zentralatom sitzen, in Lösung oder in Reinsubstanz vorbildet, wobei ein Überschuss der monomeren Vorstufe des Amins über die Stöchiometrie der gewünschten Komplexverbindung hinaus vorliegen kann. Gleichzeitig oder anschliessend, eventuell nach einem Wechsel des Lösungsmittels und/oder einem Zusatz von Vernetzern, wie $Si(OR)_4$, $R'Si(OR)_3$, $R'_2Si(OR)_2$, $Ti(OR)_4$, $R'Ti(OR)_3$, $R'_2Ti(OR)_2$, $Zr(OR)_4$, $R'Zr(OR)_3$, $R'_2Zr(OR)_2$, bzw. $Al(OR)_3$ oder $R'Al(OR)_2$, wobei R für eine 1–5 C-Atome enthaltende Alkylgruppe und R' für eine Methyl- oder Ethylgruppe steht, wird das monomere Metall-Amin-Komplexsystem bei oder unterhalb Siedetemperatur des verwendeten Solvens mit Wasser oder wässriger Säurelösung, eventuell unter gleichzeitiger oder nachfolgender destillativer Entfernung von entstehendem Alkohol, umgesetzt und dadurch polykondensiert. Das feste Polykondensat wird dann, bevorzugt bei erhöhter Temperatur, in Suspension behandelt und anschliessend durch Destillieren, Filtrieren, Zentrifugieren und/oder Dekantieren vom Lösungsmittel befreit. Dann wird es mit demselben oder einem anderen Lösungsmittel gewaschen bzw. extrahiert. Es schliesst sich der Trocknungsgang bei Temperaturen von Raumtemperatur bis 200 °C an. Gegebenenfalls kann das getrocknete Material für einen Zeitraum von 1 Stunde bis 4 Tagen bei Temperaturen von 200 bis 400 °C getempert werden. Das getemperte Produkt kann gemahlen und/oder klassifiziert werden.

Alternativ zu dieser zweistufigen Verfahrensweise kann in einigen Fällen auch überschüssiges Wasser oder überschüssige wässrige Säurelösung dem verwendeten Lösungsmittel bereits vor der in-situ-Darstellung der monomeren Komplexverbindung zugesetzt werden, da das mit trifunktionellem Silicium substituierte Amin aufgrund seiner sterischen Anordnung im Komplex schneller in koordiniertem als in unkoordiniertem Zustand polykondensiert, so dass die gewünschte polymere, unlösliche Koordinationsverbindung im Verlauf der Umsetzung quantitativ ausfällt.

Nach diesem Syntheseprinzip kann der zunächst erhaltene polymere Metall-Amin-Komplex direkt, ohne weitere Isolierung, in dem bereits vorgegebenen Lösungsmittelgemisch nach den bereits besprochenen Modifizierungsmethoden modifiziert werden und anschliessend entweder direkt seiner Verwendung zugeführt oder erst, gegebenenfalls durch Destillieren, Filtrieren und/oder Dekantieren von der flüssigen Phase abgetrennt, gewaschen bzw. extrahiert, getrocknet, gegebenenfalls getempert, gemahlen und/oder klassifiziert werden.

Prinzipiell können anstelle der Alkoxiderivate der eingesetzten Silylalkylamine oder der eingesetzten Vernetzer auch die entsprechenden Phenoxi- oder Halo-Vertreter verwendet werden, doch ist deren Herstellung teilweise aufwendiger und/oder ihr Einsatz wirkt sich wegen des bei der Hydrolyse frei werdenden Phenols bzw. Halogenwasserstoffs komplizierend aus.

Obwohl mit der Wahl des Darstellungsverfahrens und dessen Parameter Einfluss auf Oberfläche und Teilchenstruktur der Polymeren genommen werden kann, ist generell die zuerst beschriebene Darstellungsweise als die geeignetere anzusehen.

Von ihren physikalischen Eigenschaften her gesehen, verhalten sich die polymeren Metall-Amin-Komplexe gemäss der Erfindung wie spezielle Kieselsäuren oder Kieselgele und besitzen je nach Vorbehandlung spezifische Oberflächen von 0,1 bis 3000 $m^2/g$ und Teilchengrössendurchmesser von ca. 1 cm bis ca. 1 µm. An der Luft sind die Komplexe teilweise bis über 200 °C stabil. Unter Schutzgasatmosphäre liegt die thermische Stabilität noch deutlich höher, teilweise bis über 400 °C. Ihre hohe Abriebsbeständigkeit gestattet einen Einsatz nach allen gängigen Katalyse-Verfahrenstechniken.

Die erfindungsgemässen polymeren Koordinationsverbindungen des Molybdäns, Wolframs, Mangans, Rheniums und der Metalle der VIII. und I. Nebengruppe des Periodensystems stellen wertvolle Katalysatoren für chemische Umsetzungen, wie Hydroformylierungs-, Hydrierungs-, Hydrosilylierungs-, Oligomerisierungs-, Carbonylierungs-, Carboximethylierungs-, Isomerisierungs-, Metathese- und Oxidationsreaktionen sowie für Reaktionen von CO mit $H_2$ dar. Für den Fachmann ist es natürlich selbstverständlich, dass nicht alle der genannten Metalle als Katalysatoren für die gleiche Umsetzung geeignet sind, sondern dass jedes Metall bzw. jede Metall-Kombination sein spezifisches Einsatzgebiet hat.

Beispielsweise lässt sich die Hydroformylierung von Olefinen unter Verwendung der neuen polymeren Rhodium-Amin- und Kobalt-Amin-Komplexe in an sich bekannter Weise bei Wasserstoff/Kohlenmonoxid-Gesamtdrucken von 1 bis 1000 bar und Temperaturen von Raumtemperatur bis 280 °C unter oder ohne Verwendung eines Lösungsvermittlers durchführen, wobei eine hohe Katalysatoraktivität und -selektivität die ausschliessliche Bildung von Aldehyden und bereits unter relativ milden Bedingungen im Fall der Rhodium-Derivate auch die der entsprechenden Alkohole erlaubt.

Eine Hydrierung olefinischer oder acetylenischer Verbindungen kann bei Raumtemperatur oder erhöhter Temperatur, bei Unterdruck, Atmosphärendruck, oder Überatmosphärendruck durchgeführt werden. Dabei entwickeln insbesondere mit weiteren Liganden modifizierte Komplexe eine beachtliche Selektivität. Die erzielbaren Aktivitäten sind vergleichbar mit entsprechenden homogenen Katalysatorsystemen; in bezug auf die Standzeit und die Abtrennbarkeit von Bestandteilen der Reaktionsmischung, wie Lösungsmittel, Substratresten und Produkt lassen die erfindungsgemässen polymeren Komplexe jedoch erhebliche Vorteile erkennen. Nach der Abtrennung ist ein erneuter Einsatz möglich, ohne dass ein Aktivitätsverlust feststellbar ist.

Die Erfindung wird nachfolgend an Hand von Ausführungsbeispielen weiter erläutert. Die bei diesen Beispielen in den einzelnen Komplexsystemen erfindungsgemäss verwendeten speziellen polymeren Aminliganden L der Formel (1) repräsentieren einfache und leicht zugängliche Vertreter ihrer Art und besitzen demzufolge Modellcharakter. Zu dieser Aussage berechtigen insbesondere die allgemein bekannten Eigenschaften siliciumorganischer Verbindungen dieses Typs und auch die Tatsache, dass die polymeren Amine der neuen Komplexe gegenüber monomenen Aminen in ihren Lgandenqualitäten nicht wesentlich verändert sind, so dass dieses Prinzip der Polykondensation trialkoxisilylfunktioneller Amine bei geeigneter Funktionalisierung praktisch auf alle bekannten und bereits verwendeten Aminliganden

übertragen werden kann. Entsprechendes gilt natürlich auch für das verwendete Anion X.

Beispiel 1

Eine Lösung von 2,22 g (5,60 mMol) $WCl_6$ in 75 ml getrocknetem Ethanol wurde bei Raumtemperatur mit 10 g eines fein gemahlenen polymeren Amins, bestehend aus Einheiten der Formel $N(CH_2CH_2CH_2SiO_{3/2})_3$, versetzt. Diese Suspension wurde zunächst 1 Stunde bei Raumtemperatur und anschliessend 24 Stunden unter Rückfluss gerührt. Dann wurde der Feststoff abfiltriert, 4 Stunden mit trockenem Ethanol am Soxhlet extrahiert und 4 Stunden lang bei 100 °C/$10^{-1}$ mbar getrocknet. Es konnten 12,2 g (99,3% d. Theorie) eines blaugrünen Pulvers erhalten werden. Der aus Einheiten der Formel $WCl_6$ $[N(CH_2CH_2CH_2SiO_{3/2})_3]_6$ bestehende polymere Metallkomplex besass folgende Analysendaten:

| | % C | % H | % N | % W | % Cl |
|---|---|---|---|---|---|
| Theorie: | 29,81 | 5,00 | 3,86 | 8,45 | 9,78 |
| Gefunden: | 29,04 | 5,11 | 4,03 | 7,89 | 8,91 |

Beispiel 2

Eine Lösung von 1,56 g (5,71 mMol) $MoCl_5$ in 60 m getrocknetem Chloroform wurde mit 12,4 g eines polymeren Amins, bestehend aus Einheiten der Formel $(H_3C)N(CH_2CH_2CH_2SiO_{3/2})_2$ vereinigt, zunächst 1 Stunde bei Raumtemperatur und anschliessend 20 Stunden unter Rückfluss gerührt. Danach wurde der Feststoff abzentrifugiert, 3 Stunden mit Chloroform am Soxhlet extrahiert, 6 Stunden bei 80 °C/100 mbar getrocknet und anschliessend an einer Kreuzschlagmühle vermahlen. Die Ausbeute an der polymeren Molybdänverbindung mit Einheiten der Formel $MoCl_5$-$[(H_3C)N(CH_2CH_2CH_2SiO_{3/2})_2]_{10}$ betrug 13,9 g (99,5% d. Theorie).

Analysendaten:

| | % C | % H | % N | % Mo | % Cl |
|---|---|---|---|---|---|
| Theorie: | 34,36 | 6,18 | 5,72 | 3,92 | 7,24 |
| Gefunden: | 33,28 | 6,27 | 5,91 | 3,27 | 7,98 |

Beispiel 3

2,0 g (5,68 mMol) $W(CO)_6$ und 38,8 g eines polymeren Amins mit Einheiten der Formel 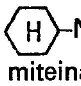$-N[(CH_2)_5SiO_{3/2}]_2$ wurden 30 Stunden lang miteinander in 250 ml siedendem Toluol zur Reaktion gebracht. Danach wurde der Feststoff von der flüssigen Phase abzentrifugiert, 6 Stunden mit Toluol extrahiert und anschliessend 4 Stunden bei 120 °C/$10^{-1}$ mbar getrocknet. Es konnten 40,5 g eines ockerfarbenen Feststoffs erhalten werden. Bei Annahme der erfolgten Bildung eines Polymersystems mit Einheiten der Formel

$W(CO)_5$ 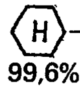$-N[(CH_2)_5SiO_{3/2}]_{2\ 20}$ betrug die Ausbeute 99,6% d. Theorie. Folgende Analysendaten wären zu erwarten gewesen:

| % C | % H | % Si | % N | % W |
|---|---|---|---|---|
| 54,55 | 8,73 | 15,70 | 3,91 | 2,57 |

Gefunden wurden:

| % C | % H | % Si | % N | % W |
|---|---|---|---|---|
| 53,89 | 8,60 | 14,94 | 4,07 | 2,31 |

Beispiel 4

3,0 g (19,87 mMol) $MnSO_4$ und 20,62 g polymeres Amin bestehend aus Einheiten der Formel $(C_8H_{17})N(CH_2SiO_{3/2})_2$ wurden 48 Stunden in 100 ml siedendem Methanol miteinander umgesetzt.

Nach Aufarbeitung der Reaktionsmischung wie nach Beispiel 1 konnten 23,3 g (98,6% d. Theorie) einer polymeren Manganverbindung mit Einheiten der Formel $MnSO_4[(C_8H_{17})N(CH_2$-$SiO_{3/2})_2]_4$ erhalten werden.

Analysendaten:

| | % C | % H | % N | % Mn | % $SO_4$ |
|---|---|---|---|---|---|
| Theorie: | 40,41 | 7,12 | 4,71 | 4,62 | 8,08 |
| Gefunden: | 39,07 | 7,51 | 5,10 | 4,55 | 7,87 |

Beispiel 5

1,2 g (3,30 mMol) $ReCl_5$ und 6,7 g polymeres Amin mit Einheiten der Formel

$$N(CH_2\overset{\overset{\displaystyle CH_3}{|}}{C}HCH_2SiO_{3/2})_3$$

wurden 20 Stunden in trockenem siedendem Ethanol gerührt. Nach Aufarbeitung der Reaktionsmischung wie nach Beispiel 1 wurden 7,69 g (97,3% d. Theorie) einer polymeren Rheniumverbindung bestehend aus Einheiten der Formel

$$ReCl_5[N(CH_2\overset{\overset{\displaystyle CH_3}{|}}{C}HCH_2SiO_{3/2})_3]_6$$

ausgewogen.

Analysendaten:

| | % C | % H | % N | % Re | % Cl |
|---|---|---|---|---|---|
| Theorie: | 36,11 | 6,06 | 3,51 | 7,77 | 7,40 |
| Gefunden: | 34,99 | 6,00 | 3,46 | 7,41 | 7,27 |

Beispiel 6

Aus 2,34 g (8,42 mMol) $FeSO_4 \cdot 7H_2O$ und 10 g polymeres Amin mit Einheiten der Formel $N(CH_2CH_2CH_2SiO_{3/2})_3$ wurden analog zu Beispiel 1 nach 24-stündiger Umsetzung in 100 ml

siedendem Methanol 11,19 g (99,3% d. Theorie) einer polymeren Eisenverbindung, bestehend aus Einheiten der Formel $FeSO_4\{N[(CH_2)_3SiO_{3/2}]_3\}_4$, hergestellt.

Analysendaten:

| | % C | % H | % N | % Fe | % $SO_4$ |
|---|---|---|---|---|---|
| Theorie: | 32,32 | 5,42 | 4,19 | 4,17 | 7,18 |
| Gefunden: | 31,28 | 5,55 | 4,95 | 3,74 | 6,80 |

Beispiel 7

Aus 0,96 g (4,03 mMol) $CoCl_2 \cdot 6H_2O$ und 29,9 g polymeres Amin mit Einheiten der Formel $N(CH_2CH_2CH_2SiO_{3/2})_3$ wurden analog zu Beispiel 1 nach 24-stündigem Rühren in 200 ml siedendem Ethanol 29,04 g (95,5% d. Theorie) eines lilafarbenen Polymerprodukts der theoretischen Zusammensetzung $CoCl_3\{N[(CH_2)_3SiO_{3/2}]_3\}_{25}$ synthetisiert.

Analysendaten:

| | % C | % H | % N | % Co | % Cl |
|---|---|---|---|---|---|
| Theorie: | 35,83 | 6,01 | 4,64 | 0,78 | 0,94 |
| Gefunden: | 34,17 | 5,98 | 5,63 | 0,78 | 0,61 |

Beispiel 8

Aus 1,6 g (6,73 mMol) $NiCl_2 \cdot 6H_2O$ und 10,5 g eines aus Einheiten der Formel

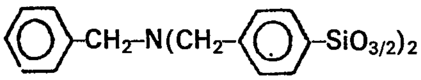

aufgebauten Amins wurden analog zu Beispiel 1 nach 18-stündigem Rühren in 100 ml refluxierendem Ethanol 11,2 (98,6% d. Theorie) einer polymeren Nickel-Amin-Verbindung, bestehend aus Einheiten der Formel $NiCl_2$

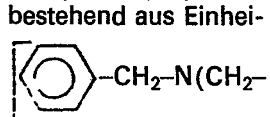

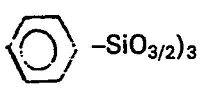

, hergestellt.

Analysendaten:

| | % C | % H | % N | % Ni | % Cl |
|---|---|---|---|---|---|
| Theorie: | 59,78 | 4,54 | 3,32 | 3,48 | 4,20 |
| Gefunden: | 59,01 | 4,77 | 3,63 | 3,09 | 3,99 |

Beispiel 9

Aus 2,0 g (6,05 mMol) $RuCl_3(CH_3CN)_3$ und 7,17 g eines aus Einheiten der Formel $N(CH_2CH_2CH_2SiO_{3/2})_3$ aufgebauten polymeren Amins wurden nach 20-stündigem Rühren in 150 ml siedendem Toluol analog zu Beispiel 1 8,40 g (99,6% d. Theorie) einer polymeren Ruthenium-Amin-Verbindung, bestehend aus Einheiten der Formel $RuCl_3[N(CH_2CH_2CH_2SiO_{3/2})_3]_4$, erhalten.

Analysendaten:

| | % C | % H | % N | % Ru | % Cl |
|---|---|---|---|---|---|
| Theorie: | 31,03 | 5,21 | 4,02 | 7,25 | 7,63 |
| Gefunden: | 30,48 | 5,37 | 3,96 | 6,85 | 6,89 |

Beispiel 10

Aus 0,225 g (0,677 mMol) $RhCl_3(CH_3CN)_3$ und 10,0 g eines polymeren Amins mit Einheiten der Formel $N(CH_2CH_2CH_2SiO_{3/2})_3$ wurden analog zu Beispiel 1 nach 24-stündigem Rühren in 150 ml siedendem Toluol 9,98 g (98,0% d. Theorie) einer polymeren Rhodium-Amin-Komplexverbindung, bestehend aus Einheiten der theoretischen Summenformel $RhCl_3\{N(CH_2CH_2CH_2SiO_{3/2})_3\}_{50}$, erhalten.

Analysendaten:

| | % C | % H | % N | % Rh | % Cl |
|---|---|---|---|---|---|
| Theorie: | 35,95 | 6,04 | 4,66 | 0,68 | 0,71 |
| Gefunden: | 34,71 | 6,24 | 4,89 | 0,71 | 0,88 |

Beispiel 11

Aus 0,466 g (1,40 mMol) $RhCl_3(CH_3CN)_3$ und 25,0 g eines polymeren, $SiO_2$-vernetzten Amins, bestehend aus Einheiten der Formel $N(CH_2CH_2CH_2SiO_{3/2})_3 \cdot SiO_2$, wurden analog zu Beispiel 10 miteinander umgesetzt. Es konnten 24,5 g (96,9% d. Theorie) einer $SiO_2$-vernetzten polymeren Rhodium-Amin-Verbindung, bestehend aus Einheiten der Summenformel $RhCl_3\{N(CH_2CH_2CH_2SiO_{3/2})_3 \cdot SiO_2\}_{50}$, erhalten werden.

Analysendaten:

| | % C | % H | % N | % Rh | % Cl |
|---|---|---|---|---|---|
| Theorie: | 29,96 | 5,03 | 3,88 | 0,57 | 0,59 |
| Gefunden: | 29,02 | 5,42 | 4,21 | 0,57 | 0,65 |

Beispiel 12

5 g einer analog Beispiel 10 hergestellten polymeren Rhodium-Amin-Komplexverbindung, bestehend aus Einheiten der Formel $RhCl_3\{N(CH_2CH_2CH_2SiO_{3/2})_3\}_5$, wurden in 70 ml trockenem Ethanol bei Raumtemperatur suspendiert. Zu dieser Suspension wurde binnen 15 min. eine Lösung von 1 g (14,69 mMol) Natriummethylat in 50 ml Ethanol zugetropft. Es wurde nach 30 min. bei Raumtemperatur und dann 4 Stunden unter Rückfluss gerührt. Anschliessend wurde der erhaltene dunkelbraune Feststoff abfiltriert, 4 Stunden mit einem Alkohol/Wasser-Gemisch (1:1) am Soxhlet extrahiert und 4 Stunden bei 80 °C/$10^{-1}$ mbar getrocknet. Es liessen sich 5,05 g

einer modifizierten polymeren Rhodium-Amin-Komplexverbindung erhalten, deren Chlorid-Gehalt gegenüber dem Ausgangswert deutlich reduziert war.

Analysendaten:

| | % C | % H | % N | % Rh | % Cl |
|---|---|---|---|---|---|
| Vor d. Behandlg. | 30,07 | 5,42 | 4,26 | 6,13 | 6,28 |
| Nach d. Behandlg. | 33,42 | 6,00 | 4,12 | 5,91 | 0,36 |

Beispiel 13

5 g einer analog Beispiel 10 hergestellten polymeren Rhodium-Amin-Komplexverbindung, bestehend aus Einheiten der Formel $RhCl_3\{N(CH_2CH_2CH_2SiO_{3/2})_3\}_{10}$, wurden unter $N_2$-Schutzgasatmosphäre in 50 ml Toluol suspendiert. Nach Zusatz von 0,826 g Triphenylphosphin wurde die Mischung anschliessend 3 Stunden unter Rückfluss gerührt. Es wurde abfiltriert, der verbleibende Feststoff 2 × mit je 30 ml Toluol gewaschen, 3 Stunden mit Ethanol am Soxhlet extrahiert und 3 Stunden bei 100 °C/$10^{-1}$ mbar getrocknet. Die erhaltenen 4,9 g polymeres Produkt besassen laut Analysen einen Phosphor-Gehalt von 0,87%, einen Rh-Gehalt von 2,83% und einen Chlorid-Gehalt von 1,34%, was auf eine Reduktion und Phosphinanlagerung schliessen lässt.

Beispiel 14

5 g einer analog Beispiel 10 hergestellten polymeren Rhodium-Amin-Komplexverbindung, bestehend aus Einheiten der Formel $RhCl_3\{N(CH_2CH_2CH_2SiO_{3/2})_3\}_{10}$, wurden in 50 ml Ethanol suspendiert.

Zu dieser auf Rückflusstemperatur erhitzten Suspension wurden zunächst 3,20 g 37%ige Formaldehyd-Lösung, verdünnt mit 10 ml Ethanol, und anschliessend 0,30 g Natriumborhydrid, gelöst in 15 ml Ethanol, zugetropft. Es wurde noch 2 Stunden unter Rückfluss gerührt, dann wurde abfiltriert und der Rückstand 4 Stunden mit Ethanol am Soxhlet extrahiert. Nach 4-stündiger Trocknung bei 100 °C/$10^{-1}$ mbar wurden 4,6 g einer modifizierten, polymeren Rhodium-Amin-Komplexverbindung erhalten. Die erzielte Modifizierung lässt sich durch nachstehende Gegenüberstellung der Chlorid-Analysen der Ausgangsverbindung und des Produkts belegen.

Analysendaten:

| | % C | % H | % N | % Rh | % Cl |
|---|---|---|---|---|---|
| Vor d. Behandlg. | 33,12 | 5,85 | 4,71 | 3,05 | 3,20 |
| Nach d. Behandlg. | 33,89 | 5,72 | 4,63 | 3,20 | 1,26 |

Beispiel 15

2,0 g (4,53 mMol) $[Rh(\overset{O}{\overset{\|}{C}}CH_3)_2]_2$ — printed as $[Rh(OC(=O)CH_3)_2]_2$ — wurden bei Raumtemperatur in 75 ml Ethanol gelöst. Nach Zugabe von 9,85 g eines polymeren Amins, bestehend aus Einheiten der Formel $(H_3C)N(CH_2CH_2CH_2SiO_{3/2})_2$, wurde dieses Gemisch zunächst 2 Stunden bei Raumtemperatur und dann 20 Stunden unter Rückfluss gerührt. Der gebildete braune Feststoff wurde abfiltriert, 4 Stunden mit Ethanol extrahiert und 12 Stunden bei 100 °C/100 mbar getrocknet. Es liessen sich 11,2 g (94,5% d. Theorie) polymerer Rhodium-Amin-Komplexverbindung, bestehend aus Einheiten der Summenformel

$$Rh(O\overset{\overset{O}{\|}}{C}CH_3)_2[(H_3C)N(CH_2CH_2CH_2SiO_{3/2})_2]_5,$$

erhalten.

Analysendaten:

| | % C | % H | % N | % Rh |
|---|---|---|---|---|
| Theorie: | 35,82 | 6,24 | 5,35 | 7,87 |
| Gefunden: | 35,07 | 6,41 | 5,87 | 7,55 |

Beispiel 16

200 g (0,317 Mol) eines monomeren Amins der Formel $N[CH_2CH_2CH_2Si(OC_2H_5)_3]_3$ und 14,85 g einer 70%igen Lösung von Tetrapropylzirkonat in Propanol wurden in 200 ml Ethanol vereinigt. Das Gemisch wurde auf 60 °C aufgeheizt und bei dieser Temperatur portionsweise mit 100 ml entsalztem Wasser versetzt. Der sich nach kurzer Zeit bildende Niederschlag wurde noch 2 Stunden unter Rückfluss gerührt, abfiltriert, mit 0,5 l Ethanol und 1 l $H_2O$ gewaschen, 5 Stunden bei 150 °C/100 bar getrocknet, 24 Stunden bei 250 °C/100 mbar getempert und anschliessend an einer Stiftmühle vermahlen. Es konnten 94,5 g (96,5% d. Theorie) eines $ZrO_2$-vernetzten Amins, bestehend aus Einheiten der Formel $N(CH_2CH_2CH_2SiO_{3/2})_3 \cdot 0,1\ ZrO_2$, erhalten werden.

Analysendaten:

| | % C | % H | % N | % Si | % Zr |
|---|---|---|---|---|---|
| Theorie: | 35,00 | 5,88 | 4,54 | 27,28 | 2,95 |
| Gefunden: | 34,76 | 6,04 | 4,90 | 26,70 | 2,79 |

10,0 g von dem gemäss vorstehender Beschreibung erhaltenen polymeren Amin wurden mit einer Lösung von 4,76 g (16,19 mMol) $Na_2PdCl_4$ in 100 ml Methanol vereinigt. Anschliessend wurde 24 Stunden unter Rückfluss gerührt. Der gebildete Feststoff wurde abfiltriert, 4 Stunden mit einem Methanol/Wasser-Gemisch (1:1) am Soxhlet extrahiert und anschliessend 5 Stunden bei 120 °C/$10^{-1}$ mbar getrocknet.

Es liessen sich 12,3 g (95,6% d. Theorie) einer polymeren, $ZrO_2$-vernetzten Palladium-Amin-Verbindung, bestehend aus Einheiten der Formel $PdCl_2\{N(CH_2CH_2CH_2SiO_{3/2})_3 \cdot 0,1\ ZrO_2\}_2$, erhalten.

Analysendaten:

| | % C | % H | % N | % Zr | % Pd | % Cl |
|---|---|---|---|---|---|---|
| Theorie: | 27,20 | 4,56 | 3,52 | 2,29 | 13,38 | 8,92 |
| Gefunden: | 26,45 | 4,41 | 3,80 | 2,04 | 12,51 | 8,59 |

Beispiel 17

3 g des nach Beispiel 16 hergestellten polymeren Palladium-Amin-Komplexes wurden in 50 ml getrocknetem Ethanol suspendiert. Im Verlauf von 15 min. wurde hierzu eine Lösung von 0,8 g Natriumborhydrid in 30 ml Ethanol zugetropft. Es wurde nach 1 Stunde bei Raumtemperatur und dann 1 Stunde unter Rückfluss gerührt. Der Feststoff wurde abfiltriert, 3 Stunden mit einem Ethanol/Wasser-Gemisch (1:1) extrahiert und 3 Stunden bei 120 °C/$10^{-1}$ mbar getrocknet.

Analysendaten:

| | % Pd | % Cl | % N |
|---|---|---|---|
| Vor Reduktion: | 12,51 | 8,59 | 3,80 |
| Nach Reduktion: | 13,20 | 0,72 | 4,21 |

Beispiel 18

Aus 1,67 g (5,62 mMol) $OsCl_3$ und 10 g eines polymeren Amins mit Einheiten der Formel $N(CH_2CH_2CH_2SiO_{3/2})_3$ wurden analog zu Beispiel 1 nach 24-stündigem Rühren in 75 ml siedendem Methanol 11,48 g (98,4% d. Theorie) einer polymeren Osmium-Amin-Verbindung, bestehend aus Einheiten der Formel $OsCl_3[N(CH_2CH_2CH_2SiO_{3/2})_3]_6$, erhalten.

Analysendaten:

| | % C | % H | % N | % Os | % Cl |
|---|---|---|---|---|---|
| Theorie: | 31,25 | 5,24 | 4,05 | 9,16 | 5,12 |
| Gefunden: | 30,55 | 5,10 | 4,26 | 8,79 | 4,97 |

Beispiel 19

Aus 2,275 g (5,39 mMol) $IrCl_3(CH_3CN)_3$ und 4,80 g eines polymeren Amins, bestehend aus Einheiten der Formel $N(CH_2CH_2CH_2SiO_{3/2})_3$, wurden analog zu Beispiel 10 nach 24-stündigem Rühren in 75 ml siedendem Toluol 6,4 g (99,9% d. Theorie) einer gelben polymeren Iridium-Amin-Verbindung, bestehend aus Einheiten der Formel $IrCl_3\{N(CH_2CH_2CH_2SiO_{3/2})_3\}_3$, erhalten.

Analysendaten:

| | % C | % H | % N | % Ir | % Cl |
|---|---|---|---|---|---|
| Theorie: | 27,30 | 4,58 | 3,54 | 16,18 | 8,95 |
| Gefunden: | 26,81 | 4,72 | 3,59 | 15,30 | 8,64 |

Beispiel 20

Aus 7,00 g (16,86 mMol) $K_2PtCl_4$ und 10,0 g eines polymeren Amins, aufgebaut aus Einheiten der Formel $N(CH_2CH_2CH_2SiO_{3/2})_3$, wurden analog zu Beispiel 16 nach 24-stündigem Rühren in 120 ml siedendem Ethanol 14,1 g (97,4% d. Theorie) einer polymeren Platin-Amin-Verbindung, aufgebaut aus Einheiten der Formel $PtCl_2[N(CH_2CH_2CH_2SiO_{3/2})_3]_2$, erhalten.

Analysendaten:

| | % C | % H | % N | % Pt | % Cl |
|---|---|---|---|---|---|
| Theoerie | 25,17 | 4,22 | 3,26 | 22,71 | 8,25 |
| Gefunden: | 25,07 | 4,39 | 3,52 | 20,98 | 8,04 |

Beispiel 21

Aus 2,875 g (16,86 mMol) $CuCl_2 \cdot 2H_2O$ und 20 g eines polymeren Amins, bestehend aus Einheiten der Formel $N(CH_2CH_2CH_2SiO_{3/2})_3$, wurden analog zu Beispiel 1 nach 24-stündigem Rühren in 150 ml siedendem Ethanol 21,4 g (96,1% d. Theorie) einer ockerfarbenen polymeren Kupfer-Amin-Verbindung, aufgebaut aus Einheiten der Formel $CuCl_2[N(CH_2CH_2CH_2SiO_{3/2})_3]_4$, erhalten.

Analysendaten:

| | % C | % H | % N | % Cu | % Cl |
|---|---|---|---|---|---|
| Theorie: | 32,75 | 5,50 | 4,24 | 4,81 | 5,37 |
| Gefunden: | 30,99 | 4,98 | 5,06 | 4,43 | 4,96 |

Beispiel 22

Aus 2,56 g (8,44 mMol) $AuCl_3$ und 10,0 g eines polymeren Amins, bestehend aus Einheiten der Formel $N(CH_2CH_2CH_2SiO_{3/2})_3$, wurden analog zu Beispiel 1 nach 24-stündigem Rühren in 120 ml siedendem Ethanol 12,2 g (97,1% d. Theorie) einer polymeren Gold-Amin-Verbindung, aufgebaut aus Einheiten der Formel $AuCl_3[N(CH_2CH_2CH_2SiO_{3/2})_3]_4$, erhalten.

Analysendaten:

| | % C | % H | % N | % Au | % Cl |
|---|---|---|---|---|---|
| Theorie: | 29,03 | 4,87 | 3,76 | 13,23 | 7,14 |
| Gefunden: | 28,84 | 4,94 | 3,89 | 12,51 | 7,02 |

Beispiel 23

Aus 2,86 g (16,84 mMol) $AgNO_3$ und 10,0 g

eines polymeren Amins, bestehend aus Einheiten der Formel $N(CH_2CH_2CH_2SiO_{3/2})_3$, wurden analog zu Beispiel 1 nach 24-stündigem Rühren in 80 ml eines auf Rückflusstemperatur erhitzten Methanol/Wasser-Gemisches (1:1) 12,5 g (97,2% d. Theorie) einer polymeren Silber-Amin-Verbindung, bestehend aus Einheiten der Formel $AgNO_3[N(CH_2CH_2CH_2SiO_{3/2})_3]_2$, erhalten.

Analysendaten:

| | % C | % H | % N ges. | % Ag | % $NO_3$ |
|---|---|---|---|---|---|
| Theorie: | 28,34 | 4,76 | 5,51 | 14,14 | 8,13 |
| Gefunden: | 27,59 | 4,83 | 5,76 | 13,95 | 7,99 |

Beispiel 24

10 g der nach Beispiel 7 hergestellten polymeren Kobalt-Amin-Verbindung, bestehend aus Einheiten der theoretischen Zusammensetzung $CoCl_2\{N[(CH_2)_3SiO_{3/2}]_3\}_{25}$, wurden zusammen mit 44,07 mg (0,133 mMol) $RhCl_3(CH_3CN)_3$ in 100 ml Toluol vereinigt. Es wurde 24 Stunden unter Rückfluss gerührt, dann der polymere Feststoff abfiltriert, 4 Stunden mit Toluol am Soxhlet extrahiert und 6 Stunden bei 150 °C/100 mbar getrocknet. Es konnten 9,9 g (98,7% d. Theorie) einer polymeren Multimetall-Amin-Verbindung, bestehend aus Einheiten der Formel 0,1 $RhCl_3 \cdot CoCl_2[N(CH_2CH_2CH_2SiO_{3/2})_3]_{25}$, erhalten werden.

Analysendaten:

| | % N | % Co | % Rh | % Cl |
|---|---|---|---|---|
| Theorie: | 4,63 | 0,78 | 0,14 | 1,08 |
| Gefunden: | 4,83 | 0,73 | 0,13 | 0,98 |

Beispiel 25

10 g einer nach Beispiel 6 hergestellten polymeren Eisenverbindung mit Einheiten der Formel $FeSO_4[N(CH_2CH_2CH_2SiO_{3/2})_3]_4$, wurden mit 2,04 g (7,47 mMol) $MoCl_5$ in 70 ml getrocknetem Chloroform vereinigt. Das Gemisch wurde 24 Stunden unter Rückfluss gerührt, dann der polymere Feststoff abfiltriert, 4 Stunden mit Chloroform extrahiert und 4 Stunden bei 100 °C/$10^{-1}$ mbar getrocknet. Es liessen sich 11,7 g (97,2% d. Th.) einer polymeren Multimetall-Amin-Verbindung, bestehend aus Einheiten der Formel $MoCl_5 \cdot FeSO_4[N(CH_2CH_2CH_2SiO_{3/2})_3]_4$, erhalten.

Analysendaten:

| | % N | % Fe | % Mo | % Cl | % $SO_4$ |
|---|---|---|---|---|---|
| Theorie: | 3,48 | 3,47 | 5,95 | 11,00 | 5,96 |
| Gefunden: | 3,59 | 3,41 | 5,78 | 10,76 | 5,81 |

Beispiel 26

2,80 g $K_2PtCl_4$, 1,837 g $RhCl_3 \cdot 3,5\ H_2O$ und 20 g eines polymeren Amins, bestehend aus Einheiten der $N(CH_2CH_2CH_2SiO_{3/2})_3$, wurden in 150 ml Ethanol vereinigt. Das Gemisch wurde 24 Stunden unter Rückfluss gerührt und dann analog zu Beispiel 1 aufgearbeitet. Es liessen sich 22,9 g (98,7% d. Theorie) einer polymeren Platin-Rhodium-Amin-Verbindung, bestehend aus Einheiten der Formel $PtCl_2 \cdot RhCl_3[N(CH_2CH_2CH_2SiO_{3/2})_3]_{10}$, erhalten.

Analysendaten:

| | % N | % Pt | % Rh | % Cl |
|---|---|---|---|---|
| Theorie: | 4,07 | 5,67 | 2,99 | 5,15 |
| Gefunden: | 4,24 | 5,39 | 2,87 | 5,02 |

Beispiel 27

Durch stufenweise Umsetzung von 10 g einer analog Beispiel 6 hergestellten polymeren Eisenverbindung, bestehend aus Einheiten der Formel $FeSO_4[N(CH_2CH_2CH_2SiO_{3/2})_3]_{15}$, mit 0,52 g $NiCl_2 \cdot 6\ H_2O$ in 100 ml Ethanol analog Beispiel 8 und des erhaltenen Produkts mit 0,59 g $MoCl_5$ analog Beispiel 25 wurden 10,6 g (97,5% d. Theorie) einer polymeren Eisen-Nickel-Molybdän-Verbindung mit Einheiten der Formel $FeSO_4 \cdot NiCl_2 \cdot MoCl_5[N(CH_2CH_2CH_2SiO_{3/2})_3]_{15}$, erhalten.

Analysendaten:

| | % N | % Fe | % Ni | % Mo | % Cl | % $SO_4$ |
|---|---|---|---|---|---|---|
| Theorie: | 4,20 | 1,12 | 1,17 | 1,92 | 4,96 | 1,92 |
| Gefunden: | 4,46 | 1,07 | 1,10 | 1,84 | 4,80 | 1,74 |

Beispiel 28

Eine Mischung von 3,022 g der gemäss Beispiel 7 hergestellten polymeren Kobalt-Amin-Verbindung mit einem Kobalt-Gehalt von 0,78%, 50 ml (0,4 Mol) Hexen-1 und 100 ml Toluol wurde in einem 500 ml Hubautoklaven einem $CO/H_2$-Kaltdruck (1:1) von 200 bar ausgesetzt. Innerhalb von 2,5 Stunden wandelten sich bei einer Temperatur von 165 °C ca. 90% des eingesetzten Hexen-1 zu n-Heptanal und 2-Methylhexanal um. Die Zusammensetzung der erhaltenen Produkte liess sich gaschromatographisch zu ca. 50% n-Heptanal, 40% 2-Methylhexanal, 1% n-Heptanol, 1% 2-Methylhexanol sowie 8% Hexan plus unumgesetztes Hexen-1 - ermitteln.

Nach dieser Umsetzung wurde der Katalysator abfiltriert, 2 × mit je 50 ml Toluol ausgewaschen und analog zu vorstehender Beschreibung erneut zur Hydroformylierung von Hexen-1 eingesetzt.

Diesmal setzten sich innerhalb von 2,5 Stunden ca. 89% des Hexen-1 zu n-Heptanal und 2-Methylhexanal um. Die Zusammensetzung der Produkte liess sich gaschromatographisch zu ca. 52% n-Heptanal, 37% 2-Methylhexanal, 2% n-Heptanol, 1% 2-Methylhexanol sowie 8% Hexan plus unumgesetztes Hexen-1 ermitteln.

Beispiel 29

7,22 g der nach Beispiel 11 hergestellten $SiO_2$-vernetzten polymeren Rhodium-Amin-Verbindung wurden zunächst 48 Stunden bei 220 °C/100 mbar im Vakuumtrockenschrank getempert. Anschliessend wurde dieses getemperte Produkt zusammen mit 300 ml Hexen-1 in einem 500 ml Hubautoklaven einem $CO/H_2$-Kaltdruck (1:1) von 200 bar und einer Temperatur von 110 °C ausgesetzt. Bei einem Druckabfall von jeweils 50 bar wurde dieser durch Aufpressen von $CO/H_2$ wieder ausgeglichen. Nach 6 Stunden Umsetzungszeit war keine Gasaufnahme mehr feststellbar. Es wurde abgekühlt, der $CO/H_2$-Überdruck abgelassen und eine gaschromatographische Untersuchung des Produktes durchgeführt. Gemäss dieser Analyse hatte eine ca. 97%ige Umsetzung des Hexen-1 - zu den gewünschten Aldehyden n-Heptanal und 2-Methylhexanal in einer Verteilung von 52:48 stattgefunden.

Anschliessend wurde die flüssige Phase vom festen Katalysatorrückstand abgetrennt und nach Zugabe von weiteren 300 ml Hexen-1 eine erneute Hydroformylierung nach vorstehendem Schema, allerdings diesmal bei einer Temperatur von 170 °C durchgeführt. Nach 7 Stunden Umsetzungszeit ergab eine GC-Untersuchung eine wiederum ca. 97%ige Hydroformylierung des Hexen-1 und eine teilweise Weiterhydrierung der gebildeten Aldehyde. Die genaue Produktverteilung betrug 3% Hexan, 6% n-Heptanal, 10% 2-Methylhexanal, 33% n-Heptanol und 48% 2-Methylhexanol.

Beispiel 30

Der nach Beispiel 17 nachbehandelte Palladium-Katalysator mit einem Gehalt von 13,20% Palladium wurde zur Hydrierung der Modellsubstanz Acrylsäureethylester eingesetzt. Hierzu wurden 32,2 mg dieses Katalysators mit 4,36 ml Acrylsäureethylester und 20 ml Toluol in einem 50 ml Glaskolben vereinigt, der mit einer Glas-Hydrierapparatur verbunden war. Unter magnetischer Rührung, einem $H_2$-Druck von ungefähr 1 bar und einer Temperatur von $80 \pm 2$ °C wurde der eingesetzte Acrylsäureethylester binnen 85 min. wie anhand der aufgenommenen Wasserstoffmenge und der gaschromatographischen Untersuchung des Reaktionsproduktes festgestellt werden konnte, quantitativ zu Propionsäureethylester umgesetzt.

Der Katalysator wurde anschliessend abfiltriert, mit $2 \times 20$ ml Toluol ausgewaschen und erneut zur Hydrierung von Acrylsäureethylester gemäss vorstehendem Schema eingesetzt, wobei eine quantitative Hydrierung in 80 min. erzielt werden konnte.

Der eingesetzte Katalysator wurde auf diese Weise insgesamt $5 \times$ abgetrennt und wieder eingesetzt, ohne dass ein Aktivitätsverlust feststellbar war.

Beispiel 31

Der nach Beisiel 20 hergestellte polymere Platin-Amin-Katalysator wurde zur Trichlorsilanaddition an Allylchlorid eingesetzt. Hierzu wurde 55,8 mg von dieser Verbindung in 24,4 ml Allylchlorid suspendiert und unter kräftigem Rühren im Verlauf von 1 Stunde bei Raumtemperatur 30,3 ml Trichlorsilan zugetropft. Anschliessend wurde die Mischung 3 Stunden unter Rückfluss erhitzt. Im Verlauf dieser Zeit kletterte die Siedetemperatur im Sumpf von 38 °C auf weit über 100 °C an. Eine NMR-spektroskopische Untersuchung der flüssigen Phase ergab, dass sie zu ca. 95% aus dem gewünschten Produkt γ-Chlorpropyltrichlorsilan bestand und nur ca. 5% unreagiertes Allylchlorid vorhanden waren.

Beispiel 32

3,23 g der nach Beispiel 24 hergestellten polymeren Rhodium-Kobalt-Amin-Verbindung wurden zunächst 48 Stunden bei 270 °C/100 mbar im Vakuumtrockenschrank getempert. Anschliessend wurde dieses getemperte Produkt zusammen mit 250 ml Hexen-1 in einem 500 ml Hubautoklaven einem $CO/H_2$-Kaltdruck (1:1) von 200 bar und einer Temperatur von 130 °C ausgesetzt. Bei einem Druckabfall von jeweils 50 bar wurde dieser durch Aufpressen von $CO/H_2$ wieder ausgeglichen. Nach 6 Stunden Umsetzungszeit war keine Gasaufnahme mehr feststellbar. Es wurde abgekühlt, der $CO/H_2$-Überdruck abgelassen und eine gaschromatographische Untersuchung des Produkts durchgeführt. Gemäss dieser Analyse hatte eine ca. 96%ige Umsetzung des Hexen-1 zu den gewünschten Aldehyden n-Heptanal und 2-Methylhexanal in einer Verteilung von 46:54 stattgefunden.

**Patentansprüche**

1. Polymere Komplexverbindungen des Molybdäns, Wolframs, Mangans, Rheniums und der Metalle der VIII. und I. Nebengruppe des Periodensystems mit kieselsäureartiger Struktur, dadurch gekennzeichnet, dass an den metallischen Zentralatomen wenigstens jeweils ein Amin der allgemeinen Formel

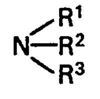

(1)

koordinativ gebunden ist, ein atomares Metall : Stickstoff-Verhältnis von 1:1 bis $1:10^6$ vorliegt, die gegebenenfalls noch freien Koordinationsstellen am Metallatom mit anderen Elektronenpaar-Donatoren besetzt sind und ein erforderlicher Ladungsausgleich mit einem anorganischen oder organischen Anion vollzogen ist, wobei in der Formel (1) $R^1$ und $R^2$ für eine Gruppe der allgemeinen Formel

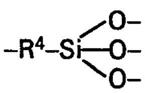 (2)

stehen, $R^4$ eine lineare oder verzweigte Alkylengruppe mit 1 bis 10 C-Atomen, eine Cycloalkylengruppe mit 5 bis 8 C-Atomen bedeutet oder für Einheiten

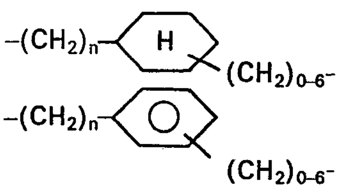 bzw.

steht, wobei n 1 bis 6 stickstoffständige Methylengruppen sind, $R^1$ und $R^2$ gleich oder verschieden sein können und die freien Valenzen der Sauerstoffatome entweder durch Siliciumatome weiterer Gruppen der Formel (2) und/oder durch vernetzende Brückenglieder

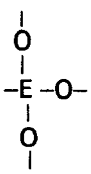 oder 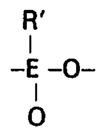 oder 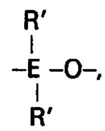,

wobei E für Silicium, Titan oder Zirkonium steht, bzw. durch Einheiten

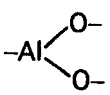 oder 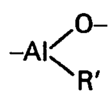

abgesättigt sind, wobei R' eine Methyl- oder Ethylgruppe ist und das Verhältnis zwischen den Siliciumatomen in (2) zu den Brückenatomen Silicium, Titan, Zirkonium, Aluminium 1:0 bis 1:10 betragen kann.

$R^3$ dieselbe allgemeine Bedeutung wie $R^1$ und $R^2$ hat oder für Wasserstoff, eine lineare oder verzweigte Alkylgruppe mit 1 bis 10 C-Atomen oder eine Cycloalkylgruppe mit 5 bis 8 C-Atomen oder die Benzylgruppe steht.

2. Polymere Komplexverbindungen nach Anspruch 1, dadurch gekennzeichnet, dass Komplexverbindungen verschiedener Metalle als Einheiten im Polymersystem nebeneinander vorliegen.

3. Polymere Komplexverbindungen nach Anspruch 1 oder 2, bestehend aus Einheiten eines oder mehrerer Komplextypen

$MoX_4L_x$, $MoX_5L_x$, $Mo(CO)_{3-5}L_x$
$WX_4L_x$, $WX_6L_x$, $W(CO)_{3-5}L_x$
$MnXL_x$, $MnX_2L_x$
$ReX_3L_x$, $ReX_4L_x$, $ReX_5L_x$, $ReX_6L_x$
$FeXL_x$, $FeX_2L_x$, $FeX_3L_x$, $Fe(CO)_{3-4}L_x$
$CoXL_x$, $CoX_2L_x$, $CoX_3L_x$, $Co_2(CO)_{6-7}L_x$
$NiXL_x$, $NiX_2L_x$, $Ni(CO)_{2-3}L_x$
$RuX_2L_x$, $RuX_3L_x$
$RhXL_x$, $RhX_2L_x$, $RhX_3L_x$
$PdX_2L_x$, $PdX_4L_x$
$OsX_4L_x$
$IrXL_x$, $IrX_3L_x$
$PtX_2L_x$, $PtX_4L_x$
$CuXL_x$, $CuX_2L_x$
$AgXL_x$
$AuXL_x$, $AuX_3L_x$

in denen als Liganden L Amine der Formel (1) vorliegen X für Chlorid, Bromid, Jodid, Nitrat, Acetat, Sulfat, Carbonat, Phosphat, Acetylacetonat oder Hydrid und x für eine Zahl von 1 bis $10^6$ steht und darüber hinaus gegebenenfalls Vernetzer gemäss Anspruch 1 zwischen den einzelnen Einheiten vorhanden sein können.

4. Mit $H_2$, CO oder $H_2$ plus CO oder mit Reduktionsmitteln oder mit Lewissäuren oder durch Einführung eines anderen Anions oder durch Reaktion mit zusätzlichen Liganden bei Gesamtdrucken von 1 bis 3000 bar und Temperaturen von –100 bis 350 °C nachbehandelte Komplexverbindungen nach Ansprüchen 1 bis 3.

5. Verfahren zur Herstellung von polymeren, in organischen Lösungsmitteln schwerlöslichen Verbindungen des Molybdäns, Wolframs, Mangans, Rheniums und der Metalle der VIII. und I. Nebengruppe des Periodensystems nach Ansprüchen 1 bis 3, dadurch gekennzeichnet, dass ein polymeres, gegebenenfalls Vernetzer enthaltendes Amin der Formel (1) mit zumindest teilweise gelösten, gegebenenfalls Chlorid, Bromid, Jodid, Nitrat, Acetat, Sulfat, Carbonat, Phosphat, Acetylacetonat oder Hydrid und/oder gegebenenfalls andere leicht verdrängbare Liganden, wie Kristallwasser, Kohlenmonoxid, Amin, Triphenylphosphin, Phosphit, Sulfid, Olefin, Acetylen, Nitril, Isonitril, Cyanat oder Isocyanat enthaltenden Verbindungen von einem oder mehreren der genannten Metalle, gegebenenfalls unter Verdrängung eines oder mehrerer Liganden, bei Raumtemperatur oder erhöhter Temperatur bis 350 °C, bei Normaldruck oder einem Überdruck, welcher der Summe der Partialdrucke der einzelnen Komponenten der Reaktionsmischung bei der jeweiligen Temperatur entspricht, umsetzt, den metallhaltigen polymeren Feststoff anschliessend durch Destillieren, Filtrieren, Zentrifugieren und/oder Dekantieren von der flüssigen Phase abtrennt, gegebenenfalls mit Wasser oder einem organischen Lösungsmittel wäscht bzw. extrahiert, nachfolgend bei Temperaturen von Raumtemperatur bis 200 °C trocknet, gegebenenfalls über einen Zeitraum von 1 Stunde bis 4 Tagen bei Temperaturen von 200 bis 400 °C tempert, anschliessend gegebenenfalls mahlt und/oder klassifiziert.

6. Verfahren nach Anspruch 5 zur Herstellung der gemischten Verbindungen nach Anspruch 2 oder 3, dadurch gekennzeichnet, dass ein polymeres, gegebenenfalls Vernetzer enthaltendes Amin der Formel (1) gleichzeitig oder nacheinander mit verschiedenen, zumindest teilweise gelösten Verbindungen der vorgesehenen Metalle nach Anspruch 5 umgesetzt wird, wobei der metallhaltige Feststoff, gegebenenfalls nach jeder Umsetzung, durch Destillieren, Filtrieren, Zentrifugieren und/oder Dekantieren von der flüssigen Phase abgetrennt, gewaschen bzw. extrahiert, getrocknet und gegebenenfalls getempert wird.

7. Verfahren nach Anspruch 5 oder 6, dadurch gekennzeichnet, dass die polymeren Komplexverbindungen, vorzugsweise suspendiert in einem Lösungsmittel, mit $H_2$, CO oder $H_2$ plus CO oder mit Reduktionsmitteln oder mit Lewissäuren oder durch Einführung eines anderen Anions oder durch Reaktion mit zusätzlichen Liganden bei Gesamtdrucken von 1 bis 3000 bar und Temperaturen von −100 bis 350 °C ein- oder mehrmalig nachbehandelt werden.

8. Verwendung der polymeren Komplexverbindungen nach vorstehenden Ansprüchen als Katalysatoren für chemische Umsetzungen, wie Hydroformylierungs-, Hydrierungs-, Hydrosilylierungs- Oligomerisierungs-, Carbonylierungs-, Carboximethylierungs-, Isomerisierungs-, Metathese- und Oxidationsreaktionen sowie für Reaktionen von CO mit $H_2$.

**Revendications**

1. Composés complexes polymères du molybdène, du tungstène, du manganèse, du rhénium et des métaux du VIII et I sous-groupes du système périodique, ayant une structure du type acide silicique, polymères caractérisés en ce que, chaque atome central métallique porte au moins une amine de formule générale:

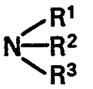 (1)

liée par coordination, qu'il existe un rapport atomique métal:azote de 1:1 à 1:10⁶, que le points de coordination éventuellement encore libres sur l'atome métallique sont occupés par d'autres paires d'électrons donneurs, l'équilibre nécessaire des charges étant complété par un anion minéral ou organique, que dans la formule (1), $R^1$ et $R^2$ représentent un groupe de formule générale:

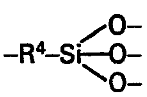 (2)

$R^4$ un groupe alcolyène linéaire ou ramifié, avec 1 à 10 atomes de C, un grupe cycloalcoylène avec 5 à 8 atomes de C, ou des groupes

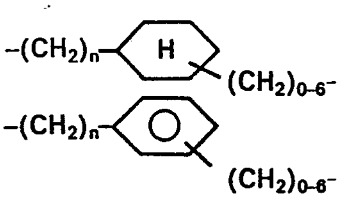

ou

où n représente 1 à 6 groupes méthylène fixés sur l'azote, $R^1$ et $R^2$ peuvent être semblables ou différents, et les valences libres des atomes d'oxygène sont saturées soit par les atomes de silicium d'autres groupes de formule (2) et/ou par des ponts réticulants du type:

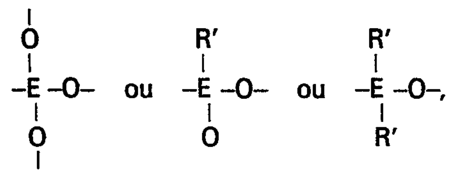

où E représente un silicium, un titane ou un zirconium, ou par des groupes:

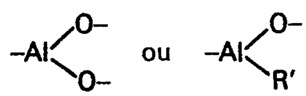

où R′ représente un groupe méthyle ou éthyle, le rapport entre l'atome de silicium dans la formule (2) et les atomes du pont silicium, titane, zirconium, aluminium, pouvant être de 1:0 à 1:10; $R^3$ a la même signification que $R^1$ et $R^2$ ou représente de l'hydrogène, un groupe alcoyle linéaire ou ramifié avec 1 à 10 atomes de C ou un groupe cycloalcoyle avec 5 à 8 atomes de C, ou un groupe benzyle.

2. Composés complexes polymères selon la revendication 1, caractérisés en ce que des composés complexes de métaux différents se trouvent côte-à-côte, sous forme de groupes unitaires dans le système polymère.

3. Composés complexes polymères selon l'une des revendications 1 ou 2, caractérisés en ce qu'ils sont constitués par des groupes d'un ou de plusieurs types de complexe:

$MoX_4L_x$, $MoX_5L_x$, $Mo(CO)_{3-5}L_x$
$WX_4L_x$, $WX_6L_x$, $W(CO)_{3-5}L_x$
$MnXL_x$, $MnX_2L_x$
$ReX_3L_x$, $ReX_4L_x$, $ReX_5L_x$, $ReX_6L_x$
$FeXL_x$, $FeX_2L_x$, $FeX_3L_x$, $Fe(CO)_{3-4}L_x$
$CoXL_x$, $CoX_2L_x$, $CoX_3L_x$, $Co_2(CO)_{6-7}L_x$
$NiXL_x$, $NiX_2L_x$, $Ni(CO)_{2-3}L_x$
$RuX_2L_x$, $RuX_3L_x$
$RhXL_x$, $RhX_2L_x$, $RhX_3L_x$
$PdX_2L_x$, $PdX_4L_x$
$OsX_4L_x$
$IrXL_x$, $IrX_3L_x$
$PtX_2L_x$, $PtX_4L_x$
$CuXL_x$, $CuX_2L_x$
$AgXL_x$
$AuXL_x$, $AuX_3L_x$

avec un ou plusieurs métaux, où les ligands L sont des amines de formule (1), où X représente un chlorure, bromure, iodure, nitrate, acétate, sulfate, carbonate, phosphate, acétylacétonate ou hydrure, et x un nombre de 1 à $10^6$, et qu'en plus, des réticulants peuvent éventuellement être présents entre les unités individuelles selon la revendication 1.

4. Composés complexes selon l'une quelconque des revendications 1 à 3, caractérisés en ce qu'ils sont traités ultérieurement avec $H_2$, CO, ou $H_2$ plus CO ou un agent réducteur, ou des acides de Lewis, ou par introduction d'un autre anion, ou par réaction avec des ligands additionnels, sous une pression totale de 1 à 3000 bars et à des températures de −100 à 350 °C.

5. Procédé pour la préparation de composés polymères, difficilement solubles dans les solvants organiques, du molybdène, du tungstène, du manganèse, du rhénium et des métaux des VIII. et I. sous-groupes du système périodique, caractérisé en ce que l'on fait réagir une amine polymère, de formule (1), contenant éventuellement un réticulant, avec des composés au moins partiellement dissous, contenant éventuellement des ligands chlorure, bromure, iodure, nitrate, acétate, sulfate, carbonate, phosphate, acétylacétonate ou hydrure et/ou éventuellement d'autres ligands faciles à déplacer, comme des eaux de cristallisation, monoxyde de carbone, amine, triphénylphosphine, phosphite, sulfure, oléfine, acétylène, nitrile, isonitrile, cyanate ou isocyanate, et qui sont des composés d'un ou plusieurs des métaux mentionnés, en chassant éventuellement un ou plusieurs ligands, à la température ambiante ou à une température élevée jusqu'à 350 °C, sous pression ordinaire ou sous une pression élevée, qui correspond à la somme des pressions partielles des composants individuels du mélange réactionnel à la température donnée, ensuite on sépare le polymère solide contenant les métaux de la phase liquide par distillation, filtration, centrifugation et/ou décantation, éventuellement on lave ou extrait avec de l'eau ou un solvant organique, ensuite on sèche à une température comprise entre la température ambiante et 200 °C, on effectue évetuellement un traitement thermique durant de 1 heure à 4 jours, à des températures comprises entre 200 et 400 °C, puis on effectue éventuellement un broyage et/ou une classification.

6. Procédé selon la revendication 5, pour l'obtention des composés mixtes selon l'une des revendications 2 et 3, caractérisé en ce que l'on fait réagir une amine de formule (1) polymérisée, contenant éventuellement un réticulant, simultanément ou successivement avec divers composés au moins partiellement dissous des métaux prévus plus haut que l'on sépare la matière solide contenant les métaux, éventuellement après chaque réaction de la phase liquide, par distillation, filtration, centrifugation et/ou décantation, qu'on la lave ou l'extrait, sèche et soumet éventuellement à un traitement thermique.

7. Procédé selon l'une des revendications 5 et 6, caractérisé en ce que les composés complexes polymères, de préférence en suspension dans un solvant, peuvent être traités ultérieurement, à une ou plusieurs reprises, avec $H_2$, CO ou $H_2$ plus $CO_2$, ou avec des agents réducteurs ou des acides de Lewis, ou par introduction d'un autre anion ou par réaction avec des ligands supplémentaires, sous une pression totale de 1 à 3000 bars et des températures de −100 à 350 °C.

8. Utilisation des composés complexes polymères selon l'une quelconque des revendications 1 à 7, comme catalyseurs de réactions chimiques telles que réactions d'hydroformylation, hydrogénation, hydrosilylation, oligomérisation, carbonylation, carboxyméthylation, isomérisation, métathèse et oxydation, ainsi que pour les réactions de CO avec $H_2$.

## Claims

1. Polymeric complexes of molybdenum, tungsten, manganese, rhenium and the metals of the VIIIth and Ist sub groups of the Periodic Table of Silicatype structure, characterised in that at least one amine of the general formula

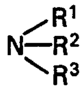 (1)

is coordinately bound in each case to the metallic central atom, the atomic metal: nitrogen ratio being from 1:1 to 1:$10^6$, the optionally still free coordination sites on the metal atom being occupied by other electron pair donors and the required charge equalization being completed with an inorganic or organic anion, wherein in formula (1) $R^1$ and $R^2$ represent a group of the general formula:

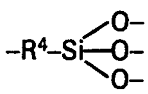 (2)

$R^4$ represents a straight or branched alkylene group having from 1 to 10 carbon atoms, a cycloalkylene group having from5 to 8 carbon atoms or the units

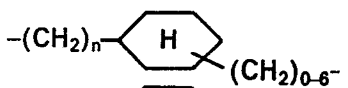 or

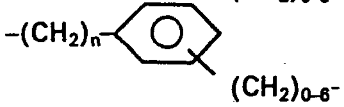

wherein n represents from 1 to 6 methylene groups bound to nitrogen, $R^1$ and $R^2$ may be the same or different and the free valences of the oxygen atoms are satisfied either by silicon atoms of further groups of the formula (2) and/or by cross-linking bridge members

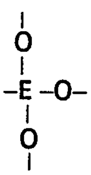 or 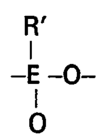 or 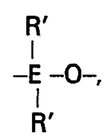,

wherein E represents silicon, titanium or zirconium, or by the units

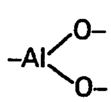 or 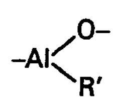

wherein R' represents a methyl or ethyl group and the ratio of the silicon atoms in (2) to the bridge atoms silicon, titanium, zirconium, aluminium may be from 1:0 to 1:10,

$R^3$ has the same definition as $R^1$ and $R^2$ or represents hydrogen, a straight or branched alkyl group having from 1 to 10 carbon atoms or a cycloalkyl group having from 5 to 8 carbon atoms or a benzyl group.

2. Polymeric complexes according to claim 1, characterised in that complexes of different metals are present as units adjacent to each other in the polymer system.

3. Polymeric complexes according to claim 1 or 2, consisting of units of one or more types of complex

$MoX_4L_x$, $MoX_5L_x$, $Mo(CO)_{3-5}L_x$
$WX_4L_x$, $WX_6L_x$, $W(CO)_{3-5}L_x$
$MnXL_x$, $MnX_2L_x$
$ReX_3L_x$, $ReX_4L_x$, $ReX_5L_x$, $ReX_6L_x$
$FeXL_x$, $FeX_2L_x$, $FeX_3L_x$, $Fe(CO)_{3-4}L_x$
$CoXL_x$, $CoX_2L_x$, $CoX_3L_x$, $Co_2(CO)_{6-7}L_x$
$NiXL_x$, $NiX_2L_x$, $Ni(CO)_{2-3}L_x$
$RuX_2L_x$, $RuX_3L_x$
$RhXL_x$, $RhX_2L_x$, $RhX_3L_x$
$PdX_2L_x$, $PdX_4L_x$
$OsX_4L_x$
$IrXL_x$, $IrX_3L_x$
$PtX_2L_x$, $PtX_4L_x$
$CuXL_x$, $CuX_2L_x$
$AgXL_x$
$AuXL_4$, $AuX_3L_x$

wherein amines of the formula (1) are present as ligands L, X represents chloride, bromide, iodide, nitrate, acetate, sulphate, carbonate, phosphate, acetylacetonate or hydride and x represents a number from 1 to $10^6$ and moreover cross-linking agents according to claim 1 can optionally be present between the individual units.

4. Complexes according to claims 1 to 3 subsequently treated with $H_2$, CO or $H_2$ plus CO or with reducing agents or with Lewis acids or by introducing another anion or by reacting with additional ligands under total pressures of from 1 to 3000 bars and at temperatures of from −100 to 350 °C.

5. A process for the production of polymeric compounds, difficultly soluble in organic solvents, of molybdenum, tungsten, manganese, rhenium and of the metals of the VIIIth and Ist sub groups of the Periodic Table according to claims 1 to 3, characterised in that a polymeric, optionally cross-linking agent-containing amine of the formula (1) is reacted with at least partially dissolved, optionally chloride, bromide, iodide, nitrate, acetate, sulphate, carbonate, phosphate, acetylacetonate or hydride and/or optionally other easily displacable ligand, such as water of crystallization, carbon monoxide, amine, triphenylphosphine, phosphite, sulphide, olefin, acetylene, nitrile, isonitrile, cyanate or isocyanate-containing compounds of one or more of the given metals, optionally while displacing one or more ligands, at room temperature or elevated temperature up to 350 °C, under normal pressure or an excess pressure which corresponds to the sum of the partial pressures of the individual components of the reaction mixture at the respective temperature, the metal-containing polymeric solid material is then separated from the liquid phase by distillation, filtration, centrifugation and/or decantation, optionally washed or extracted with water or an organic solvent, then dried at temperatures of from room temperature up to 200 °C, optionally annealed over a period of from 1 hour to 4 days at temperatures of from 200 to 400 °C, then optionally ground and/or classified.

6. A process according to claim 5 for the production of the mixed compounds according to claim 2 or 3, characterised in that a polymeric, optionally cross-linking agent-containing amine of the formula (1) is simultaneously or successively reacted with different, at least partially dissolved compounds of the prescribed metals according to claim 5, the metal-containing solid material being separated from the liquid phase, optionally after each reaction, by distillation, filtration, centrifugation and/or decantation, washed or extracted, dried and optionally annealed.

7. A process according to claim 5 or 6, characterised in that the polymeric complexes, preferably suspended in a solvent, are subsequently treated one or more times with $H_2$, CO or $H_2$ plus CO or with reducing agents or with Lewis acids or by introducing another anion or by reacting with additional ligands under total pressures of from 1 to 3000 bars and at temperatures of from −100 to 350 °C.

8. Use of the polymeric complexes according to preceding claims as catalysts for chemical reactions, such as hydroformylation, hydrogenation, hydrosilation, oligomerisation, carbonylisation, carboxymethylisation, isomerisation, metathesis and oxidation reactions, as well as for reactions of CO with $H_2$.